Europäisches Patentamt

(19) **European Patent Office**

Office européen des brevets

(11) Publication number: **0 332 679 B1**

## EUROPEAN PATENT SPECIFICATION

(45) Date of publication of patent specification: **16.06.93**  (51) Int. Cl.⁵: **A61K  31/12**

(21) Application number: **88907908.3**

(22) Date of filing: **03.08.88**

(86) International application number:
**PCT/US88/02616**

(87) International publication number:
**WO 89/01329 (23.02.89 89/05)**

(54) **ANTIVIRAL COMPOSITIONS CONTAINING AROMATIC POLYCYCLIC DIONES AND METHOD FOR TREATING RETROVIRUS INFECTIONS.**

(30) Priority: **10.08.87 US 84008**

(43) Date of publication of application:
**20.09.89 Bulletin  89/38**

(45) Publication of the grant of the patent:
**16.06.93 Bulletin  93/24**

(84) Designated Contracting States:
**AT BE CH DE FR GB IT LI LU NL SE**

(56) References cited:
**EP-A- 0 256 452**

**PROCEEDINGS OF THE NATL. ACADEMY OF
SCIENCES USA, vol. 85, no. 14, July 1988;
D.MERUELO et al., pp. 5230-5234***

**THE MERCK INDEX, 10th ed., 1983, Merck &
Co.Inc., Rahway, NJ (US); p. 710, no. 4786***

(73) Proprietor: **NEW YORK UNIVERSITY
70 Washington Square South
New York, NY 10016(US)**

Proprietor: **YEDA RESEARCH AND DEVELOP-
MENT COMPANY, LTD.
Weizmann Institute of Science Herzl Street
at Yavne Road P.O. Box 95
Rehovot 76100(IL)**

(72) Inventor: **LAVIE, David
6 Ruppin Street
76 100 Rehovot(IL)**
Inventor: **MERUELO, Daniel
9 Country Club Lane
Scarborough, NY 10510(US)**
Inventor: **LAVIE, Gad
6A Townley Road
Fairlawn, NJ 07410(US)**

Rank Xerox (UK) Business Services
(3. 10/3.6/3.3. 1)

CHEMICAL ABSTRACTS, vol. 66, no. 20, 15 May 1967, Columbus, OH (US); H. NEUMANN, no. 88613y*

DERWENT ABSTRACT, no. 304493, 1984, London (GB)*

Inventor: **REVEL, Michel, c/o Weizman Institute of Science**
**Beth Brasil 5**
**IL-76 100 Rehovot(IL)**
Inventor: **VANDE VELDE, Vincent**
**27, rue E. Hellbaut**
**B-1000 Bruxelles(BE)**
Inventor: **ROTMAN, Dalia**
**6 Alkalai Street**
**76 100 Rehovot(IL)**


(74) Representative: **Patentanwälte Grünecker, Kinkeldey, Stockmair & Partner**
**Maximilianstrasse 58**
**W-8000 München 22 (DE)**

**Description**

This invention is related to the use of at least one aromatic polycyclic dione compound in the manufacture of a medicament for the treatment of a disease caused by a retrovirus.

For many years the field of antiviral therapy has sought drugs that are capable of killing the invading pathogens without harming the host cell. The ability of viruses to physically invade a cell and to usurp the biochemical mechanisms of the cell in order to propagate their progeny, presents few unique biochemical features that can form the basis for selective inhibition of such viruses. Only a few compounds are known to possess selective antiviral activity. In particular, there are a wide variety of antiviral therapeutic agents, such as azidothymidine (AZT), dideoxycytidine, acyclovir, ribavirin, and vibaradine, which owe their selective toxicity to the fact that they can inhibit viral functions more efficiently than they can inhibit cellular functions. In general, these agents are targeted against viral polymerases, phosphorylases, and nucleotide kinases. The use of these drugs is limited due to their narrow spectrum of antiviral activity and their toxic side effects when administered systemically to a host organism over long periods of time.

Interferons are antiviral polypeptides which are currently in experimental therapeutic use in humans. However, their therapeutic value appears limited at the present time. The production and purification of human interferons require tedious procedures, the available quantities are limited and cytotoxic effects are also known to occur.

Recently it has become of great importance to find agents that are active against retroviruses, and in particular Human Immunodeficiency Virus (HIV) which is responsible for Acquired Immune Deficiency Syndrome.

Therefore, the art is constantly seeking new antiviral agents and in particular agents that are effective against retroviruses, which display high virus killing power with low cellular toxicity and have proven to be resistant to many conventional anti-viral agents.

Derwent Abstract No. 304493, 1984 discloses the isolation of an antiviral agent effective against herpes simplex virus, influenza virus, vesicular stomatitis virus, rabies virus and hepatitis B virus from the plant Hypericum erectum.

It is the object of the present invention to provide a therapeutic agent having antiviral activity and low cellular toxicity for the treatment of a disease caused by a retrovirus.

Said object is achieved by the use of at least one aromatic polycyclic dione selected from hypericin, pseudohypericin and salts thereof for the manufacture of a medicament for the treatment of a disease caused by a retrovirus.

It has been unexpectedly discovered that certain aromatic polycyclic dione compounds, exemplified by hypericin and pseudohypericin which are present in plants of the genus Hypericum (St. John's wort) and which have been isolated from the plant Hypericum triquetrifolium, are effective against retroviruses including, for example, Friend Leukemia Virus (FV), Radiation Leukemia Virus (RadLV) and human immunodeficiency virus (HIV, also known as HTLV III).

The present invention provides pharmaceutical formulations for treating mammals suffering from diseases caused by retroviruses comprising an amount effective to inhibit such viruses of hypericin, pseudohypericin or mixtures thereof. The formulations may also include physiologically-acceptable carriers and salts.

Figure 1 is a diagram showing the chemical structure of hypericin.

Figure 2 is a diagram showing the chemical structure of pseudohypericin.

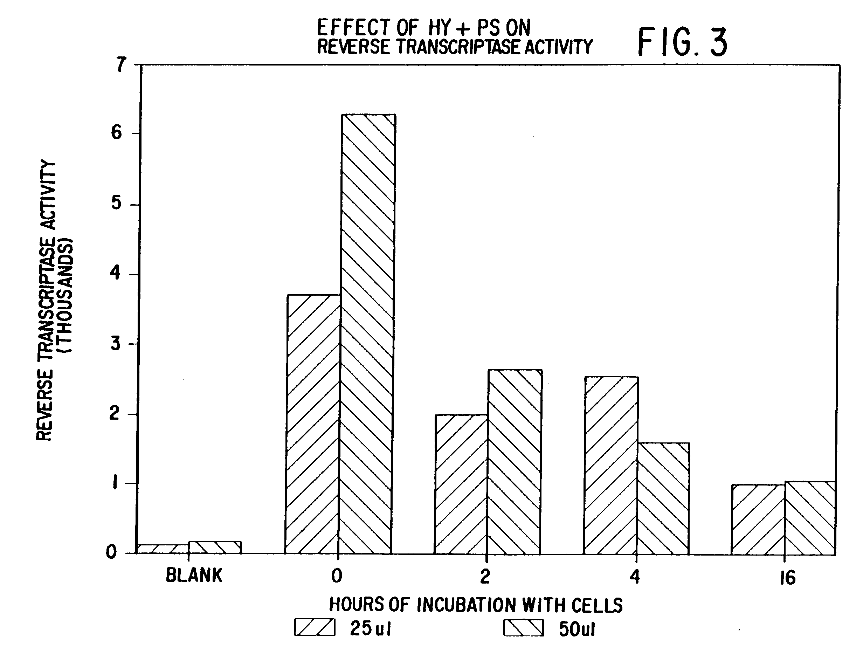

Figure 3 is a bar graph illustrating the inhibition of reverse transcriptase activity of Radiation Leukemia virus-infected mouse cells after treatment with pseudohypericin.

It has now been unexpectedly discovered that certain aromatic polycyclic diones including hypericin and pseudohypericin are antiviral agents that are highly active against retroviruses. The anti-retroviral agents are compounds which have been isolated from the perennial plant Hypericum triquetrifolium.

As used herein, the term retrovirus refers to viruses containing an RNA genome and RNA-dependent DNA polymerase (reverse transcriptase) enzymic activity. All retroviruses have common morphological, biochemical and physical properties that justify their inclusion into a single virus family. These parameters are summarized in Table A below.

TABLE A

| GENERAL PHYSICAL PROPERTIES OF KNOWN RETROVIRUSES | |
|---|---|
| Nucleic acid | linear positive-sense single-stranded RNA (60S-70S) composed of identical subunits (30S-35S); 5' structure ($m^7 G^5 ppp^5 NmpNp$); polyadenylated 3' end; repeated sequences at 3' and 5' ends; tRNA base-paired to genome complex |
| Protein | about 60% by weight; *gag,* internal structural proteins; *pol,* reverse transcriptase; *env,* envelope proteins |
| Lipid | about 35% by weight; derived from cell membrane |
| Carbohydrate | about 4% by weight; associated with *envelope* proteins |
| Physiochemical properties | density 1.16-1.18 g/ml in sucrose, 1.16-1.21 g/ml in cesium chloride; sensitive to lipid solvents, detergents, and heat inactivation (56 ° C, 30 min); highly resistant to UV- and X-irradiation |
| Morphology | spherical enveloped virions (80-120-nm diameter), variable surface projections (8-nm diameter), icosahedral capsid containing a ribonucleoprotein complex with a core shell (nucleoid) |

All retroviruses have similar overall chemical compositions. In general, they comprise about 60-70% protein, 30-40% lipid, 2-4% carbohydrate, and about 1% RNA. The envelope of retroviral particles is derived from the cell-surface membrane, and most, if not all, of the lipids in viral particles are located in the unit-membrane envelope of the virion. Examples of retroviruses include Friend Leukemia Virus (FV), Radiation Leukemia Virus (RadLV) and Human Immunodeficiency Virus (HIV).

It has now been surprisingly discovered that the aromatic polycyclic dione compounds used in the present invention inhibit the replication of Friend Leukemia virus and Radiation Leukemia virus, both in vivo and in vitro in mice. Even more surprisingly, this inhibition occurs without any significant toxicity to the recipient mammals, as shown in the examples below, wherein liver function assays, such as the levels of the enzymes lactate dehydrogenase (LDH) and serum glutamyl aminotransferase (SGOT) and those of bilirubin, which were increased due to FV infection (and are associated with the pathogenic effects of this virus) were returned to near normal values after administration of the antiviral compounds of the present invention.

The Merck Index (10th Ed. p. 710) discloses that hypericin is an antidepressant and that "small quantities appear to have a tonic and tranquilizing effect on the human organism." In addition, hypericin has been said to produce photosensitivity upon ingestion; Oxford, Raistick Biochem J. 34, 790 (1940).

Hypericin and pseudohypericin both display antiviral activity when administered to mice after retroviral infection. Hypericin was effective in inhibiting RadLV infection and pseudohypericin showed inhibitory activity against FV infection in mice as shown in Example II below. Moreover, a 50:50 mixture of hypericin and pseudohypericin led to an almost complete inhibition of the reverse transcriptase enzyme activity of RADLV-infected mouse cells in culture, as shown in Example 2 below. The same level of inhibition can be achieved by administering either hypericin or pseudohypericin alone.

The aromatic polycyclic diones appear to be capable of crossing the blood-brain barrier, as they have been previously administered as tranquilizing agents to humans. This is significant because, in the case of HIV, infection of the brain and central nervous system has been observed in infected individuals. It is also known that HIV can infect cells of the brain and central nervous system. Indeed, it has been recently found that gp120, a virally encoded polypeptide, is directly cytotoxic to cells of neuronal origin. Therefore, treatment of mammals suffering from diseases caused by these viral agents is a most important application of the present invention.

Hypericin and pseudohypericin can be used in treating mammals suffering from infections caused by retroviruses. These antiviral dione compounds are preferably obtained by extracting them from plants of the species Hypericum as detailed in Example 1 below, or alternatively may be chemically synthesized using the methods of Brockmann, M. et al, U.S. Patent No. 2,707,704, and of Brockmann, H. et al Tetrahedron Letters 23:1991-1994, 1974. Due to the wide distribution and availability of the St. John's wort plant throughout the world and the relatively convenient and inexpensive procedure for the extraction and purification of the compounds of the present invention (as detailed in Example I below), the extraction procedure is preferred when small amounts (i.e. grams) are desired. However, for the production of large scale amounts (kilograms and greater) chemical synthesis is preferred.

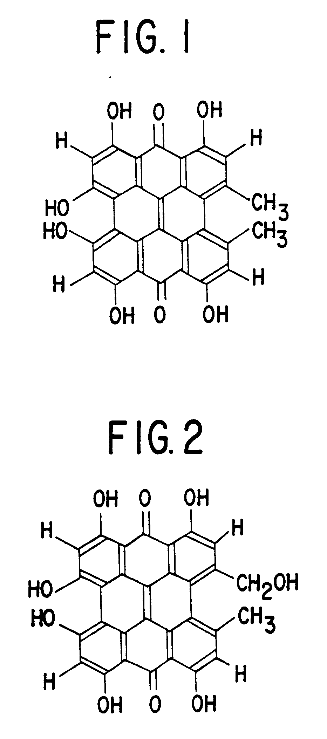

Hypericum is a genus from the family Guttifereae. It has been related also to the families Hypericaceae and Clusiaceae. The genus is geographically fairly widely distributed. It is known for its content of ether-containing oils, and for the occurrence in small glands of red fluorescent pigments, represented among others by the aromatic polycyclic structural compounds hypericin and pseudohypericin (Figure 1 and 2). Plants of the genus Hypericum have been reported to grow in very wide areas in Middle and Eastern Europe, Asia, North and South Africa. Additionally, there are reports of its occurrence in certain parts of the American continent, Australia and New Zealand. The plant is indexed in The Audubon Society Field Guide to North American Wild Flowers, Eastern Region, pg. 558, Chanticleer Press, Inc., NY.

The aromatic polycyclic diones used in the present invention can be utilized for the treatment of mammals suffering from diseases caused by retroviruses such as Acquired Immune Deficiency Syndrome (AIDS). Due to their potency and lack of cellular toxicity, the aromatic polycyclic diones will be particularly useful as specific antiviral therapeutic agents for these disorders. Currently there are no broad spectrum agents available to treat these retroviral infections without concurrent cytotoxicity.

The present invention further includes the use of salts or other derivatives of hypericin or pseudohypericin which retain their anti-viral activity. Salts in which the base is of the alkaline or amine type are particularly comprehended within the scope of the present invention.

When treating mammals suffering from infections caused by retroviruses, the determination of the most effective compound (or mixtures thereof) for treatment of the particular retrovirus responsible for the infection can be ascertained by routine experimentation using suitable in vitro systems, such as that described in Example IV below for human immunodeficiency virus or in Example II for FV and Radiation Leukemia Virus (RadLV) in experimental animals.

When employed to treat AIDS, viremia (i.e. the presence of virus in the blood stream) or sepsis (viral contamination of bodily fluids) caused by retroviruses, the aromatic polycyclic dione compounds used in the present invention may be administered orally, topically or preferably parenterally, and most preferably by intravenous administration of dosages broadly ranging between 200 $\mu$g and about 12,000 $\mu$g per kilogram (kg) of body weight per treatment and preferably between 400 and 4,000 $\mu$g per kg body weight per treatment. The duration and number of doses or treatments required to control a patient's disease will vary from individual to individual, depending upon the severity and stage of the illness and the patients condition. A typical treatment may comprise intravenous administration of from 1000 to 3000 $\mu$g per kg of body weight of one or more of the compounds used in the present invention. The total dose required for each treatment may be administered in divided doses or in a single dosage. The antiviral treatment may be administered one or two times a week, or more, as determined by the patients condition and the stage of the patients disease.

The aromatic polycyclic dione compounds used in the present invention can be incorporated in conventional, solid and liquid pharmaceutical formulations (e.g. tablets, capsules, caplets, injectable and orally administerable solutions) for use in treating mammals that are afflicted with enveloped virus infections. The pharmaceutical formulations comprise an effective antiviral amount of the aromatic polycyclic diones (as disclosed above), or mixtures thereof as individual components as at least one of the active ingredients. The quantity of effective dose supplied by each capsule, tablet or injection is relatively unimportant since the total dosage can be reached by administration of one or a plurality of capsules, tablets, injections or a combination thereof. In addition, such formulation may comprise inert constituents including pharmaceutically-acceptable carriers, diluents, fillers, salts and other materials well-known in the art depending upon the dosage form utilized. The capsules employed may be comprised of any pharmaceutically acceptable material, such as gelatin or cellulose derivatives. The tablets may be formulated in accordance with conventional procedure employing solid carriers well known in the art. Examples of solid carriers include starch, sugar, bentonite and other commonly used carriers. For example, a preferred parenteral dosage form may comprise a sterile isotonic saline solution, containing between about 0.2 (200 $\mu$g) mg per kg of body weight and about 12 mg (12,000 $\mu$g) per kg of body weight of one or more of the aromatic polycyclic diones used in the present invention or mixtures thereof. Propylene glycol, benzyl alcohol, ethanol or other biologically acceptable organic solvents may be used as diluents, carriers or solvents in the preparing solid and liquid pharmaceutical formulations containing the anti-retroviral compounds used in the present invention. Hypericin and pseudohypericin may be administered separately, or combined (mixed) in a single dosage form, or co-administered at the same time. Selection of the particular agent to be employed will be determined by ascertaining the antiviral compound or mixture that works best for a particular patient.

The aromatic polycyclic diones used in the present invention may be ideally-suited for co-administration with other agents such as immune system cells, factors such as T-cells or interleukin-2, cytotoxic agents, lymphokines such as interferons, that are known to have some effectiveness against retroviruses.

5

The present invention is described below in specific working examples which are intended to illustrate the invention.

EXAMPLE I: EXTRACTION OF HYPERICIN AND PSEUDOHYPERICIN FROM ST. JOHN'S WORT

Hypericin, ($C_{30}H_{16}O_8$, molecular weight 504.43) referred to herein as Hy, and pseudohypericin ($C_{30}H_{16}O_9$ referred to herein as Ps, molecular weight 520.43) were obtained as detailed below.

The herb of the whole St. John's wort plant was harvested at its flowering time, (July through October in the Eastern hemisphere), dried at 55°C, cut and milled, and then extracted with acetone (5-10 l/kg). One kg of the material was placed in a soxhlet (Kimax, available from Fisher Scientific, New Brunswick, NJ) and extracted until the extracting solvent was colorless (about five to ten h). The solution containing the aromatic polycyclic diones had a red fluorescent color with absorption and fluorescence spectra as described in Scheibe Schentag. Ber. 75: 2019, 1942, Brockmann, H., Natureweiss 38: 47, 1951.

The solvent (containing the aromatic polycyclic diones) was evaporated under reduced pressure to complete dryness of the residue, yielding 95 g. This residue was then further fractionated on a chromatographic column, packed with silica gel 60 (0.06-0.20mm, Malinckrodt, American Scientific Products, McGaw Park, IL). A dry chromatographic procedure was utilized whereby 25 g of the above obtained residue was dissolved in about 500 ml acetone, added to an equal amount of silica gel 60, and evaporated on a rotavapor (Buchi, American Scientific Products) with swirling until the mixture was homogeneous and dry. The mixture was then placed on top of a column containing 1 kg of silica gel 60 and eluted with chloroform until the solvent reached the bottom of the column. This was followed by washing the column with a solvent mixture comprising chloroform-acetone-methanol, 75:15:10 (Vol/Vol/Vol). When the red color became 1/5 of the original intensity, the concentration of chloroform was reduced and the column eluted with a solvent mixture comprising chloroform-acetone-methanol in the ratio of 55:15:10 respectively (Vol/Vol/Vol). Fractions of 250ml were collected. Each fraction was monitored on thin layer chromatoplates (VWR, San Francisco, CA) and the $R_f$ value of the two main red fluorescent spots under ultraviolet light at 254nm was determined. The developing solvent mixture was chloroform-acetone-methanol, 55:15:10 as above. The chromatography was completed in about two days.

Further purification and separation of the two main components was obtained by two rounds of flash chromatography using a silica gel 60 (mesh 0.04-0.06 (=0.10-0.15mm)) under pressure as described in Gas Chromatography. Principles, Techniques and Applications. A.B. Littlewood, ed. Academic Press. New York 1970.

Two main components were identified: hypericin (Hy), $R_f$ 0.45, in a yield of 0.19 g; and pseudohypericin (Ps), $R_f$ 0.35, in a yield of 0.73 g. The NMR spectrum analysis of the two components were the same as those previously reported (Brockmann, H, et al, Tetrahedron Letters 1:37, 1975).

The compounds were stored at 4°C in absolute ethanol, in the dark, until use.

EXAMPLE II: ANTIVIRAL ACTIVITY IN MAMMALS

The effects of the compounds used in the present invention on the infection of mammals with the retroviruses Friend Leukemia Virus (FV) and Radiation Leukemia Virus (RadLV) was examined as follows.

(1) Friend Leukemia Virus Infection

Friend Leukemia virus (FV) an aggressive retrovirus induces an acute erythroleukemia in sensitive strains of mice such as BALB/c and NIH SWISS mice (as described in Friend, C. J. Exp. Med. 105: 307-324, 1957; Friend, C. et al Nat. Cancer Inst. Monogr. 22: 505-522, 1966; Friend, C. et al Proc. Natl. Acad. Sci. U.S.A. 68: 378-383, 1971). The malignant transformation is the result of the combined activities of the spleen focus forming virus (SFFV) and the ecotropic murine Friend Leukemia helper virus (F-MuLv). The acute erythroleukemia is characterized by hepatosplenomegaly (a marked increase in the size of the spleen and liver) and a severe anemia.

Friend Leukemia virus was prepared by homogenizing the enlarged spleen of a mouse previously infected with FV, 10 days after intravenous virus injection. The spleen was homogenized in phosphate buffered saline in a volume equal to 10 times the weight of the isolated spleen.

The effects of Hy and Ps on the increase in spleen size (splenomegaly) of BALB/c mice (Jackson Labs, Bar Harbor, ME) was examined. In these experiments, the virus ($10^6$ focus forming units - FFU) was inoculated intravenously, and the indicated doses of the antiviral compounds of this invention were administered to the BALB/c mice intraperitoneally 24 h later. The animals were then sacrificed ten days later and their spleens weighed. The results are summarized in Table 1 below.

## TABLE 1

The effect of administering compound Ps (diluted in phosphate buffered saline with 1% ethanol) on the splenomegaly in BALB/c mice following inoculation with Friend erythroleukemia virus.

| Control Mice (PBS) | FV inoculated Mice ($10^6$ FFU) |
|---|---|
| 0.2094 Spleen weight (g ) | 1.0272 Spleen weight (g ) |
| 0.1834 Spleen weight (g ) | 0.9596 Spleen weight (g ) |
| 0.1790 Spleen weight (g ) | 1.2432 Spleen weight (g ) |
| 0.1669 Spleen weight (g ) | 1.1174 Spleen weight (g ) |
| x̄=0.1846±0.0178 | x̄=1.0865±0.1226 |
| | Net change from control=0.9019 |

| Friend Virus ($10^6$ FFU) + PS 80 μg/mouse | Friend Virus ($10^6$ FFU)+ 2 injections PS 80 μg/mouse |
|---|---|
| 0.2831 Spleen weight (g ) | 0.2457 Spleen weight (g ) |
| 0.2761 Spleen weight (g ) | 0.3400 Spleen weight (g ) |
| 0.2215 Spleen weight (g ) | 0.2938 Spleen weight (g ) |
| 0.1810 Spleen weight (g ) | 0.1956 Spleen weight (g ) |
| x̄=0.2404±0.0482 | x̄=0.2687±0.0621 |
| Net change from control=0.0558 | Net change from control=0.0841 |
| % Inhib=93.82 | % Inhib=90.70 |

| Control Mice (PBS) | Friend Mice ($2 \times 10^5$ FFU) |
|---|---|
| 0.2094 Spleen weight (g ) | 0.8911 Spleen weight (g ) |
| 0.1834 Spleen weight (g ) | 0.9211 Spleen weight (g ) |
| 0.1790 Spleen weight (g ) | 0.8004 Spleen weight (g ) |
| 0.1669 Spleen weight (g ) | 0.8662 Spleen weight (g ) |
| x̄=0.1846±0.0178 | x̄=0.8697±0.0513 |
| | Net change from control=0.6851 |

| Friend Virus ($2 \times 10^5$ FFU) + PS 80 μg/mouse | Friend Virus ($2 \times 10^5$ FFU) 2 inject PS 80 μg/mouse |
|---|---|
| 0.3457 Spleen weight (g ) | 0.4924 Spleen weight (g ) |
| 0.2784 Spleen weight (g ) | 0.2469 Spleen weight (g ) |
| 0.2208 Spleen weight (g ) | 0.2722 Spleen weight (g ) |
| 0.1791 Spleen weight (g ) | 0.2438 Spleen weight (g ) |
| x̄=0.2560±0.0723 | x̄=0.3138±0.1197 |
| Net change from control=0.0714 | Net change from control=0.1292 |
| % Inhib=89.58 | % Inhib=81.15 |

The data in Table 1 show the inhibition of splenomegaly, with median inhibition of 93.8%, following a single injection of 80 micrograms per mouse of Ps. A median inhibition of 89.6% in spleen enlargement was observed when 80 $\mu$g per mouse of Ps was administered in a single injection to mice that had previously been inoculated with 0.5ml of the virus preparation (corresponding to $2\times10^5$ FFU of virus). When two daily consecutive injections of Ps, each comprising 80 $\mu$g per mouse of the compound were administered, the median inhibition of splenomegaly was 90.7% with a viral preparation containing $10^6$ FFU and 81.7% with a viral preparation containing $2\times10^5$ FFU (Table 1).

The above results show a marked decrease in the malignant transformational capacity of the Friend Leukemia Virus (as measured by decreased splenomegaly) following the intraperitoneally administration of Ps 24 h after infection.

## (2) Co-administration with Friend Leukemia Virus

A different experimental design was also tested involving the simultaneous intravenous co-administration of Ps with the FV complex. In this case, the viral preparation was mixed with Ps at various concentrations and the mixture was injected into the mouse tail vein in a final volume of 0.5ml. The mice were sacrificed ten days later, the spleens weighed and the level of inhibition of splenomegaly subsequently determined. The results are summarized in Table 2.

### TABLE 2

The effect of intravenous co-administration of pseudo-hypericin (diluted in PBS with 1% EtOH) with FV, on viral-induced splenomegaly.

#### Spleen Weights (grams)

| | Controls | | Expt1 | Expt2 | Expt3 |
|---|---|---|---|---|---|
| PBS | PBS + 1%EtOH | FV | FV+PS 5mcg | FV+PS 20 mcg | FV+PS 50mcg |
| 0.1304 | 0.1862 | 1.1499 | 0.3425 | 0.1655 | 0.1830 |
| 0.1490 | 0.1567 | 1.0657 | 0.3766 | 0.1426 | 0.1674 |
| 0.1362 | 0.1386 | 0.9597 | 0.4005 | 0.1433 | 0.1422 |
| 0.1515 | —— | 1.1347 | 0.4255 | 0.1966 | 0.1365 |
| $\bar{x}$=0.1417 | $\bar{x}$=0.1605 | $\bar{x}$=1.0774 | $\bar{x}$=0.3862 | $\bar{x}$=0.1614 | $\bar{x}$=0.1572 |
| ±0.0101 | ±0.0240 | ±0.0866 | ±0.0353 | ±0.0253 | ±0.0217 |

% inhibition as compared to the group receiving Ps in PBS + 1% EtOH = 75.44%    100%    100%

As shown in Table 2 above, 100% inhibition of splenomegaly was found when Ps was administered with the viral complex at concentrations of 20 $\mu$g per mouse and 50 $\mu$g per mouse (average mouse weight approximately 150 grams). A mean inhibition of 75.44% was found when 5 $\mu$g per mouse was co-administered with the virus.

These results show the effectiveness of the compounds of the present invention in that as little as 5 $\mu$g per mouse was effective in inhibiting viral transformation by this aggressive RNA tumor virus.

## (3) Effect of Ps on liver function of FV infected animals

As a further demonstration of the antiviral activity of Ps, the effect of FV infection, in the presence and absence of Ps, on liver functions was monitored by analyzing the serum of infected mice for liver-associated proteins.

BALB/c mice were inoculated with FV at a concentration of $2\times10^5$ FFU. Each group of animals contained 4 mice, and analyses were done in pooled aliquots.

TABLE 3

| EFFECT OF FRIEND VIRUS WITH OR WITHOUT PS (IN PBS WITH 1% EtOH) ON LIVER FUNCTIONS | | | | | | | | | |
|---|---|---|---|---|---|---|---|---|---|
| | CHOLEST mg/dl | Total PROTEIN g/dl | ALBUMIN g/dl | BU mg/dl | AP IU/l | LDH IU/l | SGOT IU/l | SGPT IU/l | GGT miu/ml |
| PBS | 122 | 5.7 | 3.3 | 0.2 | 226 | 1075 | 132 | 105 | 3 |
| IVFV | 118 | 5.5 | 3.4 | 0.4 | 200 | 6710 | 450 | 110 | 4 |
| IVFVIPPS | 109 | 5.7 | 3.4 | 0.1 | 226 | 3303 | 213 | 99 | ND |
| IPFV | 111 | 5.4 | 3.3 | 0.2 | 223 | 3330 | 245 | 100 | 3 |
| IPFVIVPS | 137 | 5.5 | 3.2 | 0.3 | 200 | 2305 | 244 | 135 | 4 |

PBS - Phosphate Buffered Saline

IVFV - Intravenous administration of Friend Virus

IVFVIPPS - Intravenous administration of Friend Virus, intraperitoneal injection of Ps compound

IPFV - Intraperitoneal administration of Friend Virus

IPFVIVPS - Intraperitoneal administration of Friend Virus, intravenous injection of Ps compound

In Table 3, BU = Total Bilirubin, AP = Alkaline phosphatase, LDH = Lactate dehydrogenase, SGOT = Serum glutamyl aminotransferase, SGPT = Serum glutamyl peptidyltransferase, and GGT = Gamma-glutamyl-transpeptidase.

As shown in Table 3, intravenous inoculation with FV led to an increase in LDH, SGOT and SGPT enzyme activity and to an increased total serum bilirubin accumulation in infected mice. Such increases are indicators of liver function damage, well-known in the art. Administration of Ps, both intravenously and intraperitoneally, led to a reduction in these virally-induced increases.

(4) Antiviral Effect on Radiation Leukemia Virus Infection (RadLV)

The intrathymic inoculation of murine radiation leukemia virus in susceptible strains of mice results in the malignant transformation of the thymocytes and the development of overt leukemia (as described in Meruelo, D. et al J. Exp. Med. 147: 470-487, 1978). This transformation has been shown to be associated with the major histocompatibility complex (MHC) which determines resistance or susceptibility to the leukemogenic effects of the virus. Among the early events which follow the viral infection in some mouse strains is a dramatic increase in the cell surface expression of class I H-2 antigens. This increase is then followed by a reduction in the expression of these antigens after the malignant transformation and subsequent development of leukemia. The changes in H-2 antigen expression have been utilized to monitor viral infectivity and to determine the effects of Hy and Ps on the level of infectivity of this virus.

Murine radiation leukemia virus (RadLV) was inoculated intrathymically into B10.T(6R) mice as described in Meruelo et al (supra). Groups of six mice were treated 24 h later with 30 $\mu$g per mouse of compound Hy administered intraperitoneally in isotonic saline solution. The mice were sacrificed ten days later and the expression of Class I H-2 antigens determined by analysis with a fluorescence activated cell sorter (Ortho Cytofluorograph Model 50H, Ortho Diagnostic Systems, Westwood, MA) (FACS), using X-56 anti-H-2D[d] mouse monospecific antibodies (as described in Meruelo, et al supra; similar antibodies are available as ATCC No. HB75, American Type Culture Collection, Rockville, MD). The results are shown in Table 4 below.

## TABLE 4

**The effect of Hy and Ps on the increase in H-2 antigen expression in thymocytes after intrathymic inoculation of mouse radiation leukemia virus in B10.T(6R) mice.**

| Base Line (untreated mice) | | Virus Induced (inoculated mice) | | 30 $\mu g$/mouse Hy (inoculated with RadLV and Hy) | | 30 $\mu g$/mouse PS | |
|---|---|---|---|---|---|---|---|
| % of cells stained with antibody | mean fluorescent intensity in fluorescent units | % of cells stained with antibody | mean fluorescent intensity in fluorescent units | % of cells stained with antibody | mean fluorescent intensity in fluorescent units | % of cells stained with antibody | mean fluorescent intensity in fluorescent units |
| 94.1 | (606.8) | 97.3 | (752.9) | 76.7 | (625.5) | 97.4 | (713.3) |
| 76.0 | (515.7) | 91.7 | (599.0) | 77.2 | (596.9) | 94.3 | (639) |
| 45.1 | (440.3) | 92.5 | (599.6) | 96.3 | (723.6) | | |
| 76.0 | (518.7) | 94.0 | (703.1) | 84.8 | (663.5) | | |
| 89.5 | (596.7) | 96.3 | (729.2) | 54.0 | (513.6) | | |
| 87.4 | (558.8) | 97.3 | (719.9) | 97.8 | (657.3) | | |
| $\bar{x}$= 78.02 | | $\bar{x}$=94.85 | | $\bar{x}$=81.13 | | | |

$$(1) \ \% \ \text{inhibition} = 100 - \left[ \frac{(\text{Treated } 30 \ \mu g) - (\text{Base Line})}{\text{Virus Induced} - \text{Base Line}} \right] \times 100 = 82\%$$

As can be seen from the data in Table 4, Hy exerted a significant inhibition in the increase in H-2 expression in four of the six mice tested (Table 4), indicating a significant reduction in the infective capacity of the virus following the intraperitoneal administration of compound Hy. This occurred as late as 24 h after the initial viral inoculation. No effects on H-2 expression induced by RadLV were found with compound Ps.

EXAMPLE III: EFFECTS OF PS AND HY ON RNA-DEPENDENT DNA POLYMERASE (REVERSE TRANSCRIPTASE)

Retroviruses (RNA tumor viruses) carry within the virion the reverse transcriptase enzyme which, upon infection of the cell with the virus, recruits the cellular synthetic machinery and transcribes a complementary DNA (cDNA) copy of the virion RNA using the viral RNA genome as a template. The determination of levels of reverse transcriptase (RT) activity in the growth medium of cells infected with retroviruses is a well-known method with which to evaluate the release of infectious virus particles by cells and has been used to measure the antiviral activity of the components of the present invention. The effect of a mixture of both Hy and Ps at a concentration of 10 $\mu$g per ml (of each compound) on the RadLV RT activity was examined.

The mixture of the two compounds was administered 2, 4 and 16 h post-infection and the supernatants were harvested and assayed for RT activity according to the method of Stephenson et al, Virology 48:749-756, 1972, and Weissbach et al, J. Virol. 10:321-327, 1972.

The assay was performed as follows:

a) Preparation of Virus

RadLV-infected lymphoblastoid cells, line B10.T(6R) were pelleted by centrifugation at 4°C, 3500 rpm for 15 min. The top 2/3 of the supernatant (10ml) was removed for assay. The supernatant was then centrifuged at 40,000 rpm for 1 h at 4°C in 12ml ultracentrifuge tubes, using a fixed-angle ultracentrifuge rotor (Ti70 rotor, Beckman Instruments, Fullerton, CA). The supernatant was carefully decanted and the tubes dried. The pellet was resuspended in 200 microliters of a buffer containing 0.02M Tris/HCl, pH 7.8, 0.1 M NaCl, 0.001M dithiothreitol, and 0.2% Triton X-100. The mixture of the buffer and the virus-containing pellet was vortexed and incubated on ice for 30 min before use.

The reverse transcriptase assay was performed in a volume of 100 $\mu$l containing the following components:

TABLE 5

| Reagent Stock | $\mu$l of Stock per assay | final concentration per assay (100 $\mu$l) |
|---|---|---|
| sol'n A: 0.5 M Tris/HCl pH 7.8(37°) | | 50 mM |
| 0.6 M KCl | 10 ul | 60 mM |
| sol'n B: 2.0 mM Mn Acetate | 10 ul | 0.2 mM |
| sol'n C: 40 mM Dithiothreitol (DTT) | 5 ul | 2 mM |
| Triton X-100 (10%) | 1 ul | 0.1% |
| poly(rA).(dT)$_{12}$ (10 A$_{260}$ U/ml)[1] | 4 ul | 0.4 A$_{260}$/ml (20 $\mu$g/ml) |
| dTTP (2 x 10$^{-4}$ M) | 10 ul | 2 x 10$^{-5}$ M |
| $^{3}$H-dTTP (500$\mu$Ci/ml)[2] | 10 ul | 5 uCi |
| 2500 $\mu$Ci/ml | 50 ul | |

[1] obtained from Pharmacia Fine Chemicals, Piscataway, NJ

[2] obtained from New England Nuclear, Boston, MA

50 $\mu$l of the reaction mixture was mixed with 50 $\mu$l of the above obtained supernatant and incubated for 1 h at 30°C. The reaction was stopped by the addition of 0.1ml of 0.06 M sodium pyrophosphate, vortexed and incubated on ice. 2ml of 10% trichloroacetic (TCA) containing 0.3 M sodium pyrophosphate was added, the mixture vortexed and incubated on ice for 10-20 min. TCA-precipitable radioactivity was determined after filtrations of samples using glass fiber filters (2.4cm Whatman GFC, Whatman, Clifton, NJ). The results are shown in Figure 2.

The data in Figure 2 shows that incubation of virally-infected cells with the 50:50 mixture of Hy and Ps led to at least a 50% decrease in the detectable RT activity at 2 and 4 h post infection and further inhibited this enzyme approximately 75% when assayed 16 h post-infection.

EXAMPLE IV: INHIBITION OF HIV BY THE COMPOUNDS used in THE PRESENT INVENTION

The activity of Ps, Hy and mixtures thereof against human immunodeficiency virus (HIV) may be investigated in the following manner. HIV-infected, OKT4+ lymphoblastoid cells, such as clone H9 (described in Popovic, M., et al, Science 224:497-500, 1984) is maintained in RPMI-1640 medium (GIBCO, Grand Island, New York) containing 20% fetal calf serum (Flow Laboratories, Inglewood, CA). Triplicate cultures of cells, seeded at a concentration of about 4x10$^5$ cells per ml, are exposed to polybrene (2 $\mu$g per ml, Sigma Chemical Co., St. Louis, MO), infected with 2x10$^8$ HTLV III particles per 4x10$^5$ H9 cells, and cultured in the presence or absence of Ps, Hy and mixtures thereof as in Example 2 above.

The antiviral activity of the compounds used in the present invention is determined by monitoring the reverse transcriptase activity and the expression of HIV proteins p24 and p17, as described in Sarin, P.S. et al, J. Nat. Cancer Inst. 78:663-665, 1987.

EXAMPLE V: Expression of HTLV III gag proteins p24 and p17.-

H9 cells (2x10$^5$), either uninfected or HTLV III infected, are continuously exposed to various concentrations of Ps, Hy and mixtures thereof at concentrations between 5 and 100 $\mu$g per ml for 4 days. The percentage of cells expressing p24 and p17 gag proteins of HTLV III is determined by indirect immunofluorescence microscopy with the use of mouse monoclonal antibodies to HTLV-III p17 and p24 (available in numerous commercial sources such as those in HIV serum antigen detection kits from Abbott Labs, North Chicago, IL, and from Du Pont, Wilmington, DE). The positive cells are visualized by treatment with fluorescein-labeled goat anti-mouse IgG (Cappell Laboratories, Cochranville, PA). The experiments are performed in duplicate (at least three times).

EXAMPLE VI: Determination of reverse transcriptase activity

Uninfected H9 or H9 cells infected with HTLV-III (500 virus particles/cell) are exposed to various concentrations of Ps, Hy and mixtures thereof as above. At day 4, supernatants of the cultures are collected and virus particles are precipitated with polyethylene glycol and obtained by centrifugation as described above in Example 2 for FV. The virus pellet is suspended in 300 $\mu$l of buffer containing 50 mM Tris-HCl (pH 7.5), 5 mM dithiothreitol, 250 mM KCl, and 0.25% Triton X-100. Reverse transcriptase activity in these samples is analyzed in a 50-$\mu$l reaction mixture containing 50 mM Tris-HCl (pH 7.5), 5mM dithiothreitol, 100 mM KCl, 0.01% Triton X-100, 10 $\mu$l dT$_{15}$rA$_n$ as template primer, 10 mM MgCl$_2$, 15 $\mu$M [$^3$H]dTTP (New England Nuclear, Boston, MA), and 10 $\mu$l of disrupted virus suspension. After incubation for 1 h at 37°C and subsequent addition of 50 $\mu$g of yeast tRNA (Sigma Chemical, St. Louis, MO), the incorporation into cold trichloroacetic acid-insoluble fraction is assayed as in Example 2 above. Assays are performed in duplicate and repeated three times.

## Claims

1. Use of at least one aromatic polycyclic dione selected from hypericin, pseudohypericin and salts thereof for the manufacture of a medicament for the treatment of a disease caused by a retrovirus.

2. The use of claim 1 which comprises a parenteral dosage form.

3. The use of claim 1 which comprises a solid oral dosage form selected from a tablet or a capsule.

4. The use of any of claims 1 to 3 in which said treatment involves administration of between 200 $\mu$g and 12,000 $\mu$g of said at least one compound per kg of body weight of a mammal to be treated.

5. The use of claim 1 comprising a pharmaceutically acceptable carrier or diluent.

6. The use of claim 1 which comprises an injectable solution.

7. The use of claim 1 wherein said virus comprises Friend Leukemia virus.

8. The use of claim 1 wherein said virus comprises Radiation Leukemia virus.

9. The use of claim 1 wherein said virus comprises human immunodeficiency virus.

## Patentansprüche

1. Verwendung wenigstens eines aromatischen polycyklischen Dions, gewählt aus Hypericin, Pseudohypericin und Salzen davon, zur Herstellung eines Medikaments zur Behandlung einer Krankheit, verursacht durch einen Retrovirus.

2. Verwendung nach Anspruch 1, welche eine parenterale Dosierungsform umfaßt.

3. Verwendung nach Anspruch 1, welche eine feste, orale Dosierungsform, gewählt aus einer Tablette oder einer Kapsel, umfaßt.

4. Verwendung nach einem der Ansprüche 1 bis 3, worin die Behandlung die Verabreichung von 200 $\mu$g bis 12000 $\mu$g der wenigstens einen Verbindung pro Kilogramm Körpergewicht eines zu behandelnden Säugers beinhaltet.

5. Verwendung nach Anspruch 1, umfassend einen pharmazeutisch annehmbaren Träger oder Verdünnungsmittel.

6. Verwendung nach Anspruch 1, welche eine injizierbare Lösung umfaßt.

7. Verwendung nach Anspruch 1, worin das Virus Friend-Leukämie-Virus umfaßt.

8. Verwendung nach Anspruch 1, worin das Virus Strahlungsleukämie-Virus umfaßt.

9. Verwendung nach Anspruch 1, worin das Virus humanes Immundefizienz-Virus umfaßt.

**Revendications**

1. Utilisation d'au moins une dione polycyclique aromatique choisie parmi l'hypericine, la pseudohypericine et leurs sels pour la fabrication d'un médicament pour le traitement d'une maladie provoquée par un rétrovirus.

2. Utilisation suivant la revendication 1, qui comprend une forme d'administration parentérale.

3. Utilisation suivant la revendication 1, qui comprend une forme d'administration orale solide choisie parmi un comprimé ou une gélule.

4. Utilisation suivant l'une quelconque des revendications 1 à 3, dans laquelle ce traitement implique l'administration d'une quantité comprise entre 200 $\mu$g et 12.000 $\mu$g de ce composé au moins présent par kg de poids corporel d'un mammifère à traiter.

5. Utilisation suivant la revendication 1, qui comprend un support ou un diluant acceptable du point de vue pharmaceutique.

6. Utilisation suivant la revendication 1, qui comprend une solution injectable.

7. Utilisation suivant la revendication 1, dans laquelle ce virus est le virus de la leucémie de Friend.

8. Utilisation suivant la revendication 1, dans laquelle ce virus est le virus de la leucémie des radiations.

9. Utilisation suivant la revendication 1, dans laquelle ce virus est le virus de l'immunodéficience humaine.

# FIG. 1

# FIG. 2

EFFECT OF HY + PS ON
REVERSE TRANSCRIPTASE ACTIVITY     FIG. 3